# EUROPÄISCHE PATENTSCHRIFT

(11) **EP 2 146 838 B1**
(45) Veröffentlichungstag und Bekanntmachung des Hinweises auf die Patenterteilung: **29.03.2017**
(21) Anmeldenummer: 08734951.0
(22) Anmeldetag: 02.04.2008
(51) Int. Cl.: B29C 49/42, A61L 2/08, A61L 2/10, B65B 55/08

(54) **BEHÄLTERHERSTELLUNGSVORRICHTUNG UND HERSTELLVERFAHREN FÜR FORMKÖRPER**
APPARATUS FOR THE PRODUCTION OF RECEPTACLES, AND METHOD FOR THE PRODUCTION OF MOLDED ARTICLES
DISPOSITIF DE PRODUCTION DE RÉCIPIENTS ET PROCÉDÉ DE PRODUCTION DE CORPS MOULÉS

(30) Priorität: 13.04.2007 DE 102007017938
(43) Veröffentlichungstag der Anmeldung: 27.01.2010
(73) Patentinhaber: KHS GmbH, 44143 Dortmund (DE)
(72) Erfinder: TILL, Volker, 65719 Hofheim am Taunus (DE)
(74) Vertreter: Hausfeld, Norbert
(86) Internationale Anmeldenummer: PCT/EP2008/002602
(87) Internationale Veröffentlichungsnummer: WO 2008/125216

(56) Entgegenhaltungen:
- WO-A-03/100116
- WO-A1-97/18154
- DE-T2- 69 624 509
- FR-A1- 2 815 542
- JP-A- H10 167 226
- JP-A- H11 137 645
- JP-A- 2005 008 243
- US-A1- 2005 118 057
- US-B1- 6 562 281
- US-B1- 6 818 068

## Beschreibung

Die Erfindung betrifft eine Behälterherstellungsvorrichtung zum Herstellen von Kunststoffbehältern aus Vorformlingen (Preforms), insbesondere Flaschen, kleine Fässchen (Kegs) und sonstige Behälter aus PET, die eine Blasmaschine und geeignete Transportvorrichtungen umfasst, wobei an der Blasmaschine oder auf dieser mindestens ein Strahlungsemitter angebracht ist und/oder ein Strahlungsemitter auf mindestens einen Teilbereich der Blasmaschine gerichtet ist. Weiterhin ist ein entsprechendes Sterilisierungsverfahren für Blasmaschinen von der Erfindung umfasst.

Im Stand der Technik sind Behälterherstellungsvorrichtungen bekannt, die mittel einer Streckblasmaschine aus Vorformlingen, zum Beispiel Flasche formen. DE 10 2005 015 565 A1, DE 10 2004 061 230 A1 oder DE 295 08 864 U1 beschreiben solche Behälterherstellungsvorrichtungen.

Zunehmend besteht das Erfordernis, immer höhere Reinheiten oder aseptische Bedingungen bei der Herstellung und Abfüllung von Behältern und Flaschen zu erreichen. Üblich ist es, dass bereits der Vorformling von möglichen Anhaftungen gereinigt wird, indem zum Beispiel ein Strom ionisierter Luft ins Innere geleitet wird. DE 101 40 906 B4, EP 0 895 816 A1 oder DE 199 09 488 A1 beschreiben derartige Vorrichtungen und Verfahren. Weiterhin sind aus WO 01/31680 A1 oder DE 102 36 683 A1 Vorrichtungen und Verfahren bekannt, bei welchen Flaschen beschichtet und gleichzeitig sterilisiert werden, indem unter Vakuum ein Plasma gezündet wird. Weiter Vorrichtungen und Verfahren sind bekannt. Für Füllereinheiten sind nass- und trockenaseptische Verfahren bekannt.

Aus der US 2005/0118057 A1, die den Oberbegriff der Ansprüche 1, 11 offenbart, und der US 6,562,281 B1 ist bekannt, Vorformlinge oder Preformen vor der Einleitung in die eigentliche Blasmaschinen und insbesondere vor der Aufheizstrecke mittels Strahlung oder mit H2O2 zu sterilisieren.

Nachteilig an diesem bekannten Stand der Technik ist, dass diese Behandlungsschritte nur vor oder nach der Blasmaschine durchgeführt werden können, da die Blasmaschine selbst bisher nicht steril betrieben werden kann.

Aufgabe der Erfindung ist es daher, ein steriles Gesamtverfahren vorzuschlagen. Diese Aufgabe wird erfindungsgemäß durch Merkmale der Behälterherstellungsvorrichtung gemäß Anspruch 1 und die Merkmale des Verfahrens gemäß Anspruch 11 gelöst. Vorteilhafte Ausführungen sind in den Unteransprüchen genannt.

Die Behälterherstellungsvorrichtung zum Herstellen von Kunststoffbehältern, insbesondere Flaschen, kleinen Fässchen und sonstige Behälter aus PET, aus Preformen, umfasst eine Blasmaschine und geeignete Transportvorrichtungen, wobei im Nahbereich vor, an der Blasmaschine oder auf dieser, mindestens ein Strahlungsemitter angebracht ist und/oder ein Strahlungsemitter auf mindestens einen Teilbereich der Blasmaschine oder deren Umhausung gerichtet ist. Der Strahlungsemitter ist idealerweise ein Elektronenstrahler oder UV-Strahler. Als UV-Strahler ist einer für gepulstes UV-Licht besonders geeignet. Es ist möglich je nach Bestrahlungsaufgabe mehreren gleiche oder verschiedenartige Strahlungsemitter zu verwenden. Somit ist es möglich, mindestens einen Teil der Oberflächen der Behälterherstellungsvorrichtung und/oder den die Behälterherstellungsvorrichtung umgebenden Raum zu sterilisieren und/oder permanent steril zu halten.

Eine vorteilhafte Ausgestaltung besteht darin, bei einer Rotationsblasmaschine Strahlungsemitter auf oder an mindestens einem der rotierenden Elemente anzubringen, so dass dieser mit dem Kreisel der Blasmaschine umläuft. Auf diese Weise kann die Anzahl der Emitter verringert und die Strahlungsverteilung im Raum erhöht werden. Dabei kann mindestens ein Strahlungsemitter auf dem die Blasformen tragenden oder zuführenden Karussell beziehungsweise Sternrad angebracht sein, welcher im vorgesehenen Betrieb mit diesem Karussell oder Sternrad rotiert.

Eine weitere Verbesserung besteht darin, mindestens einen Teil der Oberflächen der Behälterherstellungsvorrichtung elektrostatisch aufladbar auszubilden, um so im bestimmungsgemäßen Betrieb die Elektronenstrahlen zu lenken. Dabei werden idealerweise Teile der Oberflächen der Behälterherstellungsvorrichtung, insbesondere der Blasmaschine elektrostatisch positiv aufladbar ausgebildet, um so Elektronen und/oder Elektronenstrahlen durch den Potentialunterschied anzuziehen. Auf diese Weise können zum Beispiel schlecht zugängliche Fläche ebenfalls mit dem Elektronenstrahl beaufschlagt werden.

In einer Weiterbildung der Behälterherstellungsvorrichtung wird stromaufwärts der Behälterherstellungsvorrichtung eine Sterilisierungseinheit vorgesehen, in welcher die Preformen stromaufwärts der Blasmaschine mittels Strahlungsemitter sterilisiert werden. Hierzu kann die Sterilisierungseinheit einen oder mehrere Strahlungsemitter, insbesondere Elektronenstrahler umfassen, welche in die Öffnung des Vorformlings einführbar sind.

Vorteilhafterweise werden die Behälterherstellungsvorrichtung und die Sterilisierungseinheit direkt miteinander verbunden, so dass diese einen gemeinsamen, geschlossenen Raum bilden. Weiterhin kann stromabwärts der Behälterbehandlungsvorrichtung eine Füllereinheit angeordnet sein, die direkt und abdichtende mit der Behälterherstellvorrichtung verbunden ist, so dass in dieser Ausführungsform Sterilisiereinheit für Preformen, Behälterherstellvorrichtung und Füllereinheit in einer geblockten Bauweise einen im Wesentlichen gemeinsamen Raum bilden. In analoger Weise kann stromabwärts der Behälterbehandlungsvorrichtung eine Füllereinheit angeordnet sein, ohne dass eine Sterilisierungseinheit für Preformen vorgesehen ist, wobei die Füllereinheit direkt mit der Behälterherstellvorrichtung verbunden ist und diese so einen gemeinsamen Raum bilden.

Von der Erfindung ist auch ein Herstellverfahren für Kunststoffbehälter aus Preformen umfasst, bei der eine Behälterherstellungsvorrichtung gemäß Anspruch 1 eingesetzt wird. Als Kunststoffbehälter kommen insbesondere Flaschen, kleine Fässchen (Kegs) und sonstige Behälter aus PET oder anderen geeigneten Kunststoffen in Frage.

Idealerweise wird dabei die Behälterherstellvorrichtung mit einer Sterilisierungseinheit für Vorformlinge und/oder einer Füllereinheit als gemeinsamer Sterilraum betrieben. Eine Verbesserung des Verfahrens besteht darin, dass im Übergangsbereich zwischen der Füllereinheit, Behälterherstellvorrichtung und/oder der Sterilisierungseinheit für Preformen eine oder mehrer Schleusen und/oder zusätzliche Sterilisierungsschritte vorgesehen sind. Eine besondere Ausführungsvariante besteht darin, dass ein Teil der Vorrichtungen und/oder der Behälter mit einem Nassverfahren sterilisiert werden. Idealerweise wird der Bereich mit einem Nassverfahren sterilisiert, dessen oberes Ende tiefer als die Öffnungen der Vorformlinge und/oder Behälter liegt und sich nach unten bis zum Boden erstreckt.

- Fig. 1:: Darstellung der Behälterherstellungsvorrichtung in einer Draufsicht
- Fig. 2:: Darstellung einer Sterilisierungseinheit für Vorformlinge, einer Behälterherstellungsvorrichtung und einer Fülleinheit in einer Draufsicht

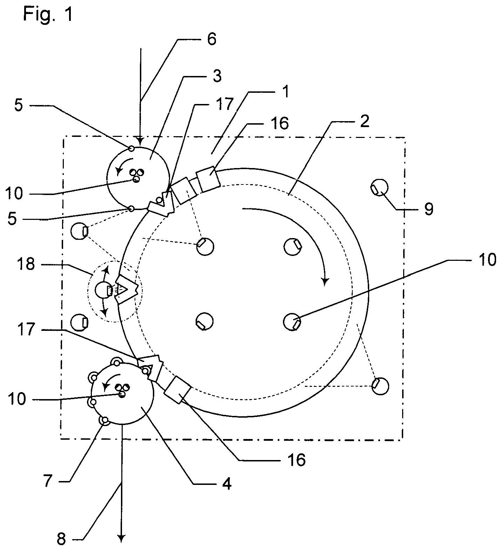

Die mit einer strichpunktierten Linie umrahmte Behälterherstellvorrichtung 1 umfasst ein Karussell 2, einen Zulaufstern 3 und einen Auslaufstern 4. Weitere Details, wie Heizvorrichtung, Blasformen etc. sind bekannt und in Fig. 1 nicht dargestellt. Die Vorformlinge 5 und die geformten Flaschen 7 werden vorrangig hängend transportiert, wozu diese in bekannter Weise durch Greifer oder Halterungen am Hals gehalten werden ("Neck Handling"). Die Vorformlinge 5 werden über den Förderweg 6 an den Zulaufstern 3 herangeführt und von diesem auf das Karussell 2 übergeben. Auf dem Karussell 2 sind Kavitäten 16 auf dem Umfang angeordnet, die aus Gründen der Übersichtlichkeit nur teilweise dargestellt sind und in denen der Streck-/Blasvorgang in bekannter Weise durchgeführt wird. Gegenüber dem Zulaufstern 3 ist einen geöffnete Kavität 17 gezeigt, ebenso gegenüber dem Auslaufstern 4, welcher die fertig geblasen Flasche übernimmt.

Nach dem Blasvorgang an der Rundläuferblasmaschine werden die fertig geformten Flaschen 7 vom Auslaufstern 4 übernommen und aus der Behälterherstellungsvorrichtung über den Förderweg 8 herausgeführt. In der Behälterherstellvorrichtung 1 sind eine Vielzahl von Strahlungsemittern angeordnet. Die statischen Strahlungsemitter 9 sind am Rand der Behälterherstellvorrichtung 1 angeordnet und strahlen in Richtung der Einbauten. Einige Strahlungsemitter sind so ausgerichtet, dass diese auf die, die Umhausung bildenden Wände inklusive der Decke und dem Boden gerichtet sind. Die Strahlungsrichtung ist durch eine Strichpunktierte Linie angedeutet.

Auf dem Zulaufstern 3 und auf dem Auslaufstern 4 sowie auf dem zentralen Karussell 2 sind jeweils mehrere Strahlungsemitter 10 angeordnet, die gemeinsam mit dem Stern beziehungsweise dem Karussell umlaufen. Auf diese Weise wird eine intensive und mehrfach gestreute Strahlung erzeugt.

In dem gezeigten Beispiel sind nur Elektronenstrahler vorgesehen, wobei eine Kombination mit zum Beispiel gepulsten UV-Lampen sinnvoll ist. Die mit einer strichpunktierten Linie umrundete Station 18 dient der Innensterilisation der Kavität 17. Hierbei ist der Strahler besonders nah gegenüber dem Karussell 2 angeordnet und kann eine gewisse Wegstrecke dem Karussell 2 beziehungsweise der geöffneten Kavität 17 folgen, um den Sterilisierungseffekt im Inneren der Kavität 17 zu verbessern.

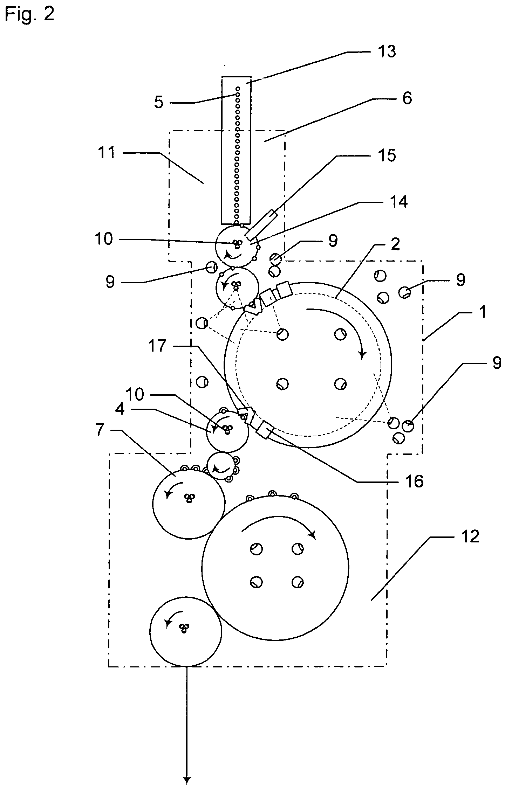

Fig. 2 zeigt die in Fig. 1 gezeigte und vorstehend beschriebene Behälterherstellvorrichtung 1 in einer gemeinsamen Einhausung mit einer Sterilisierungseinheit 11 für Vorformlinge 5 und einem Füllereinheit 12. Diese so genannte geblockte Bauweise hat den großen Vorteil, dass sie sehr kompakt und damit kostengünstiger ist, da Zwischenreinigungs- und einige Transportschritte entfallen. Die Beschriftungen haben, sofern nicht etwas Gegenteiliges gesagt ist, die identische Bedeutung, wie in Fig. 1.

Über eine nicht näher dargestellte Heiß- und Zuführeinheit 13 für die Vorformlinge 5, gelangen diese in die Sterilisierungseinheit 11, wo sie von Behandlungsstern 14 übernommen werden. Am Umfang des Behandlungssterns 14 ist eine Sterilisierungsvorrichtung 15 angeordnet. Zur Sterilisierung eines Vorformlings 5 wird eine Sterilisierungssonde, an deren Umfang und/oder Spitze mindestens ein Strahlungsemitter angeordnet ist, in den Vorformling eingeführt. Hierzu sind Strahlungsdichten von 15 bis 30 kJ/kg erforderlich.

In dem dargestellten Ausführungsform sind im Bereich der Sterilisierungseinheit 11 sind stromabwärts der Sterilisierungsvorrichtung 15 beidseitig zum Übergabebereich vom Behandlungsstern 14 zum Zuführungsstern 3 der Behälterherstellvorrichtung 1 statische Strahlungsemitter 9 angeordnet. An die Behälterherstellvorrichtung 1 schließt sich als weitere Einheit desselben Blocks die Fülleinheit 12 an. Hier sind ebenfalls statische Strahlungsemitter 9 und rotierende Strahlungsemitter 10 vorgesehen.

Die Behälterherstellvorrichtung 1 und das Verfahren ist beispielhaft beschrieben, wobei viele Varianten nach demselben Prinzip möglich sind. Insbesondere kann zwischen Sterilisierungseinheit und Behälterherstellvorrichtung eine Vorheißeinheit für Vorformlinge angeordnet sein kann, wenn die Vorformlinge der Sterilisierungseinheit kalt zugeführt werden.

## Patentansprüche

1. Behälterherstellungsvorrichtung (1) zum Herstellen von Kunststoffbehältern (7) aus Preformen (5), insbesondere Flaschen, kleine Fässchen und sonstige Behälter aus PET, umfassend eine Blasmaschine und geeignete Transportvorrichtungen, wobei an der Blasmaschine oder auf dieser mindestens ein Strahlungsemitter (10) angebracht ist und/oder ein Strahlungsemitter (10) auf mindestens einen Teilbereich der Blasmaschine und/oder mindestens auf eine Teilfläche der inneren Oberflächen der die Behälterherstellvorrichtung (1) umschließende Einhausung gerichtet ist, **dadurch gekennzeichnet, dass** die Blasmaschine eine Rotationsblasmaschine ist und Strahlungsemitter (10) derart auf oder an mindestens einem der rotierenden Elemente angebracht ist, dass dieser mit dem Element umläuft, und wobei mindestens ein Strahlungsemitter (10) auf dem die Blasformen tragenden oder zuführenden Karussell (2) beziehungsweise Sternrad (3, 4) angebracht ist, welcher im bestimmungsgemäßen Betrieb mit diesem Karussell (2) oder Sternrad (3, 4) umläuft.

2. Behälterherstellungsvorrichtung (1) nach Anspruch 1, **dadurch gekennzeichnet, dass** der mindestens eine Strahlungsemitter (10) ein Elektronenstrahler oder UV-Strahler ist, wobei der UV-Strahler idealerweise ein gepulster UV-Strahler ist.

3. Behälterherstellungsvorrichtung nach einem der Ansprüche 1 oder 2, **dadurch gekennzeichnet, dass** mehrere gleiche oder unterschiedliche Strahlungsemitter (9, 10) vorgesehen sind.

4. Behälterherstellungsvorrichtung (1) nach einem der Ansprüche 1 bis 3, **dadurch gekennzeichnet, dass** mindestens ein Teil der Oberflächen der Behälterherstellungsvorrichtung (1), insbesondere der Blasmaschine elektrostatisch aufladbar ausgebildet ist, um so Elektronenstrahlen zu lenken.

5. Behälterherstellungsvorrichtung (1) nach einem der Ansprüche 1 bis 4, **dadurch gekennzeichnet, dass** mindestens ein Teil der Oberflächen der Behälterherstellungsvorrichtung (1), insbesondere der Blasmaschine elektrostatisch positiv aufladbar ausgebildet ist, um so Elektronen und/oder Elektronenstrahlen anzuziehen.

6. Behälterherstellungsvorrichtung (1) nach einem der Ansprüche 1 bis 5, **dadurch gekennzeichnet, dass** stromaufwärts der Behälterherstellungsvorrichtung (1) eine Sterilisierungseinheit (11) vorgesehen ist, in welcher die Preformen (5) stromaufwärts der Blasmaschine mittels Strahlungsemitter (10) sterilisiert werden.

7. Behälterherstellungsvorrichtung (1) nach einem der Ansprüche 1 bis 6, **dadurch gekennzeichnet, dass** die Sterilisierungseinheit (11) für Preformen (5) Strahlungsemitter umfasst, welche in die Öffnung der Preform (5) einführbar sind.

8. Behälterherstellungsvorrichtung (1) nach einem der Ansprüche 1 bis 7, **dadurch gekennzeichnet, dass** die Sterilisierungseinheit (11) direkt mit der Behälterherstellvorrichtung (1) verbunden ist und so einen gemeinsamen Raum bilden, wobei zwischen Sterilisierungseinheit (11) und Behälterherstellvorrichtung (1) eine Heißeinheit für Vorformlinge angeordnet sein kann.

9. Behälterherstellungsvorrichtung (1) nach Anspruch 8, **dadurch gekennzeichnet, dass** stromabwärts der Behälterbehandlungsvorrichtung (1) eine Füllereinheit (12) angeordnet ist, wobei die Füllereinheit (12) direkt mit der Behälterherstellvorrichtung (1) verbunden ist und Sterilisiereinheit (11) für Preformen (5), Behälterherstellvorrichtung (1) und Füllereinheit (12) einen im Wesentlichen gemeinsamen Raum bilden.

10. Behälterherstellungsvorrichtung (10) nach einem der Ansprüche 1 bis 7, **dadurch gekennzeichnet, dass** stromabwärts der Behälterbehandlungsvorrichtung (1) eine Füllereinheit (12) angeordnet ist, wobei die Füllereinheit (12) direkt mit der Behälterherstellvorrichtung (1) verbunden ist und diese so einen gemeinsamen Raum bilden.

11. Herstellverfahren für Kunststoffbehälter (7) aus Preformen (5), insbesondere Flaschen, kleine Fässchen und sonstige Behälter aus PET, **dadurch gekennzeichnet, dass** eine Behälterherstellungsvorrichtung (1) nach einem der vorstehenden Ansprüche eingesetzt wird, um mindestens einen Teil der Oberflächen der Behälterherstellungsvorrichtung (1) und/oder die mindestens einen Teil der inneren Oberflächen der die Behälterherstellvorrichtung (1) umschließenden Einhausung zu sterilisieren und/oder permanent steril zu halten.

12. Herstellverfahren nach Anspruch 11, **dadurch gekennzeichnet, dass** eine Behälterherstellungsvorrichtung (1) nach einem der Ansprüche 8 bis 10 verwendet wird, wobei der eine gemeinsame Raum als Sterilraum betrieben wird.

13. Herstellverfahren nach Anspruch 12, **dadurch gekennzeichnet, dass** im Übergangsbereich zwischen der Füllereinheit (12), Behälterherstellvorrichtung (1) und/oder der Sterilisierungseinheit (11) für Preformen (5) eine oder mehrere Schleusen und/oder zusätzliche Sterilisierungsschritte vorgesehen sind.

14. Herstellverfahren nach einem der Ansprüche 11 bis 13, **dadurch gekennzeichnet, dass** ein Teil der Vorrichtungen und der Behälter mit einem Nassverfahren sterilisiert werden, wobei insbesondere der Bereich mit einem Nassverfahren sterilisiert wird, dessen oberes Ende tiefer als die Öffnungen der Vorformlinge und/oder Behälter (7) liegt und sich nach unten bis zum Boden erstreckt.

## Claims

1. A receptacle production apparatus (1) for the production of plastic containers (7) made of preforms (5), more particularly bottles, kegs and other containers that are made of PET, comprising a blow moulding machine and appropriate transport devices,
wherein at least one radiation emitter (10) is attached to or on this blow moulding machine and/or a radiation emitter (10) is oriented towards at least a partial area of the blow moulding machine and/or at least towards a partial area of the inner surfaces of the housing enclosing the apparatus (1) for the production of receptacles,
**characterised in that**
- the blow moulding machine is a rotary blow moulding machine and a radiation emitter (10) is attached on or to at least one of the rotating elements such that said radiation emitter (10) is rotating with the element, and wherein at least one radiation emitter (10) is attached to the carousel (2) or star wheel (3, 4), respectively, carrying or supplying the blow moulds, said radiation emitter (10) rotating with this carousel (2) or star wheel (3, 4) while operation is carried out as intended.

2. The apparatus (1) for the production of receptacles in accordance with Claim 1, **characterised in that** the at least one radiation emitter (10) is an electron radiation emitter or UV radiation emitter wherein, ideally, the UV radiation emitter is a pulsed UV radiation emitter.

3. The apparatus for the production of receptacles in accordance with any one of Claims 1 or 2, **characterised in that** a plurality of identical or different radiation emitters (9, 10) is provided.

4. The apparatus (1) for the production of receptacles in accordance with any one of Claims 1 to 3, **characterised in that** at least a portion of the surfaces of the apparatus (1) for the production of receptacles, more particularly of the blow moulding machine, is formed to be imposed with an electrostatic charge in order to guide electron beams in this manner.

5. The apparatus (1) for the production of receptacles in accordance with any one of Claims 1 to 4, **characterised in that** at least a portion of the surfaces of the apparatus (1) for the production of receptacles, more particularly the blow moulding machine, is formed to be imposed with a positive electrostatic charge in order to attract electrons and/or electron beams in this manner.

6. The apparatus (1) for the production of receptacles in accordance with any one of Claims 1 to 5, **characterised in that** a sterilisation unit (11) is provided upstream of the apparatus (1) for the production of receptacles, with the preforms (5) being sterilised in said sterilisation unit (11) by means of radiation emitters (10) upstream of the blow moulding machine.

7. The apparatus (1) for the production of receptacles in accordance with any one of Claims 1 to 6, **characterised in that** the sterilisation unit (11) for preforms (5) comprises radiation emitters which can be introduced into the opening of the preform (5).

8. The apparatus (1) for the production of receptacles in accordance with any one of Claims 1 to 7, **characterised in that** the sterilisation unit (11) is directly connected to the apparatus (1) for the production of receptacles such that they form a common space, wherein a hot unit for preforms can be arranged between the sterilisation unit (11) and the apparatus (1) for the production of receptacles.

9. The apparatus (1) for the production of receptacles in accordance with Claim 8, **characterised in that** a filler unit (12) is arranged downstream of the apparatus (1) for the production of receptacles, wherein the filler unit (12) is directly connected to the apparatus (1) for the production of receptacles, and the sterilisation unit (11) for preforms (5), the apparatus (1) for the production of receptacles and the filler unit (12), in essence, form a common space.

10. The apparatus (1) for the production of receptacles in accordance with any one of Claims 1 to 7, **characterised in that** a filler unit (12) is arranged downstream of the apparatus (1) for the production of receptacles, wherein the filler unit (12) is directly connected to the apparatus (1) for the production of receptacles such that they form a common space.

11. A method for the production of plastic containers (7) made of preforms (5), more particularly bottles, kegs and other containers that are made of PET, **characterised in that** an apparatus (1) for the production of receptacles in accordance with any one of the preceding claims is inserted in order to sterilise and/or maintain in a permanently sterile state at least a portion of the surfaces of the apparatus (1) for the production of receptacles and/or at least a portion of the inner surfaces of the housing enclosing the apparatus (1) for the production of receptacles.

12. The production method in accordance with Claim 11, **characterised in that** use is made of an apparatus (1) for the production of receptacles in accordance with any one of Claims 8 to 10, wherein the common space is operated as a sterile space.

13. The production method in accordance with Claim 12, **characterised in that** one or a plurality of locks and/or additional sterilisation steps are provided in the transition area between the filler unit (12), the apparatus (1) for the production of receptacles and/or the sterilisation unit (11) for preforms (5).

14. The production method in accordance with any one of Claims 11 to 13, **characterised in that** a portion of the apparatuses and the containers is sterilised using a wet method, wherein a wet method is used to sterilise in particular the area the upper end of which is located at a lower level than the openings of the preforms and/or containers (7) and extends downwards to the bottom.

## Revendications

1. Dispositif (1) de production de récipients pour la production, à partir de préformes (5), de récipients en matière plastique (7), notamment de bouteilles et flacons, de petits fûts et d'autres récipients en PET, comportant une machine de moulage par soufflage et des dispositifs de transport appropriés, un émetteur de rayonnement (10) au moins étant installé sur ou contre la machine de moulage par soufflage et/ou un émetteur de rayonnement (10) étant dirigé vers au moins un secteur de la machine de moulage par soufflage et/ou au moins une partie des surfaces intérieures du carter entourant le dispositif de production de récipients (1), **caractérisé en ce que** la machine de moulage par soufflage est une machine de moulage par soufflage rotative et qu'un émetteur de rayonnement (10) est installé sur ou contre au moins un des éléments en rotation de façon à tourner avec cet élément, un émetteur de rayonnement (10) au moins étant installé sur le carrousel (2) ou la roue à étoile (3, 4) qui transporte ou amène les moules de soufflage, cet émetteur tournant avec ce carrousel (2) ou cette roue à étoile (3, 4) lors du fonctionnement normal du dispositif.

2. Dispositif de production de récipients (1) selon la revendication 1, **caractérisé en ce que** l'émetteur ou l'un des émetteurs de rayonnement (10) est un émetteur d'électrons ou de rayons UV, l'émetteur de rayons UV étant, dans l'idéal, un émetteur de rayons UV pulsé.

3. Dispositif de production de récipients selon l'une des revendications 1 ou 2, **caractérisé en ce que** plusieurs émetteurs de rayonnement (9,10) identiques ou différents sont prévus.

4. Dispositif de production de récipients (1) selon l'une des revendications 1 à 3, **caractérisé en ce qu**'une partie au moins des surfaces du dispositif de production de récipients (1), et notamment de la machine de moulage par soufflage, est conçue pour pouvoir être chargée électrostatiquement de façon à diriger ainsi des faisceaux d'électrons.

5. Dispositif de production de récipients (1) selon l'une des revendications 1 à 4, **caractérisé en ce qu**'une partie au moins des surfaces du dispositif de production de récipients (1), et notamment de la machine de moulage par soufflage, est conçue pour pouvoir être chargée avec une charge électrostatique positive de façon à attirer ainsi des électrons et/ou des faisceaux d'électrons.

6. Dispositif de production de récipients (1) selon l'une des revendications 1 à 5, **caractérisé en ce qu**'en amont du dispositif de production de récipients (1) est prévue une unité de stérilisation (11) dans laquelle les préformes (5) sont stérilisées au moyen d'émetteurs de rayonnement (10) en amont de la machine de moulage par soufflage.

7. Dispositif de production de récipients (1) selon l'une des revendications 1 à 6, **caractérisé en ce que** l'unité de stérilisation (11) des préformes (5) comporte des émetteurs de rayonnement pouvant être introduits dans l'ouverture de la préforme (5).

8. Dispositif de production de récipients (1) selon l'une des revendications 1 à 7, **caractérisé en ce que** l'unité de stérilisation (11) est directement reliée au dispositif de production de récipients (1) pour former ainsi un espace commun, une unité de chauffage pour les préformes pouvant être agencée entre l'unité de stérilisation (11) et le dispositif de production de récipients (1).

9. Dispositif de production de récipients (1) selon la revendication 8, **caractérisé en ce qu**'en aval du dispositif de traitement de récipients (1) est agencée une unité de remplissage (12), l'unité de remplissage (12) étant directement reliée au dispositif de production de récipients (1) et l'unité de stérilisation (11) des préformes (5), le dispositif de production de récipients (1) et l'unité de remplissage (12) formant un espace essentiellement commun.

10. Dispositif de production de récipients (10) selon l'une des revendications 1 à 7, **caractérisé en ce qu**'en aval du dispositif de traitement de récipients (1) est agencée une unité de remplissage (12), l'unité de remplissage (12) étant directement reliée au dispositif de production de récipients et formant ainsi un espace commun avec celui-ci.

11. Procédé de production, à partir de préformes (5), de récipients en matière plastique (7), notamment de bouteilles et flacons, de petits fûts et d'autres récipients en PET, **caractérisé en ce qu**'un dispositif de production de récipients (1) selon l'une des revendications précédentes est utilisé pour stériliser et/ou maintenir en permanence la stérilité d'une partie au moins des surfaces du dispositif de production de récipients (1) et/ou une partie au moins des surfaces intérieures du carter entourant le dispositif de production de récipients (1).

12. Procédé de production selon la revendication 11, **caractérisé en ce qu**'un dispositif de production de récipients (1) selon l'une des revendications 8 à 10 est utilisé, l'espace commun faisant fonction d'espace stérile.

13. Procédé de production selon la revendication 12, **caractérisé en ce qu**'un ou plusieurs sas et/ou une ou plusieurs étapes de stérilisation supplémentaires sont prévues dans la zone de transition entre l'unité de remplissage (12), le dispositif de production de récipients (1) et/ou l'unité de stérilisation (11) des préformes (5).

14. Procédé de production selon l'une des revendications 11 à 13, **caractérisé en ce qu**'une partie des dispositifs et des récipient sont stérilisés par un procédé humide, le procédé humide étant notamment appliqué à la zone dont le bord supérieur est situé plus bas que les ouvertures des préformes et/ou récipients (7) et s'étend vers le bas jusqu'au niveau de leur fond.
